# EUROPÄISCHE PATENTANMELDUNG

(11) **EP 0 717 941 A2**
(43) Veröffentlichungstag der Anmeldung: **26.06.1996**
(21) Anmeldenummer: 95107516.7
(22) Anmeldetag: 17.05.1995
(51) Int. Cl.: A43B 17/02, A43B 7/14

(54) **Orthopädische Einlage**

(30) Priorität: 21.12.1994 DE 9420397 U; 23.12.1994 DE 9420643 U; 31.01.1995 DE 29501471 U
(71) Anmelder: IPOS GMBH & CO. KG., D-21337 Lüneburg (DE)
(72) Erfinder: Prahl, Jan, D-21379 Rullstorf (DE)
(74) Vertreter: Richter, Werdermann & Gerbaulet

(57) **Zusammenfassung**

Die orthopädische Einlage (100) mit einer ebenen Unterseite (101) und einer ein Fußbett (110) oder Fersenbett (10) bildenden, ausgearbeiteten Oberseite (102) ist mit mindestens einem Polster (20;20') im Bereich druckempfindlicher Stellen des Fußes, insbesondere des Fersensporns, versehen, wobei die Einlage (100) und das Polster (20;20';30) aus einem Kunststoff, insbesondere einem Silikonkautschuk, bestehen, daß das Polster durch Ausbildung eines Bereichs mit einer in der Unterseite der Einlage (100) in dem das Polster bildenden Bereich ausgebildeten konkaven Einziehung bzw. Ausnehmung (23, 123) in dem Formkörper der Einlage (100) erhalten wird, wobei dieser polsterartige Bereich aus einem Material mit einer Shore-Härte besteht, die gleich oder ungleich der Shore-Härte des Einlagenmaterials ist.

## Beschreibung

Die Erfindung betrifft eine orthopädische Einlage mit einer ebenen Unterseite und einer ein Fußbett oder Fersenbett bildenden, ausgearbeiteten Oberseite mit mindestens einem eingearbeiteten Polster im Bereich druckempfindlicher Stellen des Fußes, insbesondere des Fersensporns, wobei die Einlage und das Polster aus einem Kunststoff, insbesondere einem Silikonkautschuk bestehen.

Durch das DE-GM 87 00 681 ist eine orthopädische Einlage oder Fußbettung mit einer im wesentlichen ebenen, einem Innenschuh angepaßten Unterseite und einer ein Fußbett bildenden, ausgearbeiteten Oberseite mit eingearbeiteten Polstern im Bereich druckempfindlicher Stellen des Fußes bekannt, bei der in ein Grundmaterial geringerer Elastizität Polster aus Silikonkautschuk eingesetzt bzw. unterlegt oder aufgelegt sind. Mit einer derartigen Einlage sollen sich insbesondere bei großflächigen Polstern angenehme, verbesserte Trageigenschaften erzielen lassen, die bei druckempfindlichen Veränderungen oder Erkrankungen am Fuß eine wesentliche Schmerzerleichterung aufgrund der spezifischen Eigenschaften des Silikonkautschuks herbeiführen. Dadurch, daß das eingesetzte Polstermaterial ein verbessertes Tragverhalten durch hohe Rückstellkräfte und gute Stoßabsorptionen aufweist, ist diese orthopädische Einlage als stützendes oder korrigierendes Hilfsmittel bei erworbenen oder angeborenen Fehlbildungen des Fußes und überall dort einsetzbar, wo eine Schmerzlinderung und gute Druckverteilung, z.B. bei entzündlichen Erkrankungen des Fußes, gewünscht werden.

Die Herstellung von Fersenkissen aus Silikonkautschuk ist durch "Orthopädietechnik", 11/87, S.654 bis 655, bekannt. Ein derartiges Fersenkissen dient zur Versorgung des Rückfußes im Fersenbereich und insbesondere dort, wo eine Dämpfung im Fersenbereich oder bei plantarem Fersensporn gewünscht wird. Dabei werden die Eigenschaften des Silikonkautschuks verwendet, der eine gleichmäßige Druckverteilung ermöglicht, Stoßbelastungen abfängt und somit hohe Dämpfungseigenschaften besitzt.

Das DE-GM 78 34 409 beschreibt ein Fußbett, insbesondere eine Einlegesohle, für die orthopädische Behandlung von Fußbeschwerden mit einem Träger, der den anatomischen Verhältnissen des Fußes angepaßt ist, wobei auf dem Träger eine Polsterschicht aus einem nachgiebigen Material angeordnet ist. Diese Polsterschicht besteht aus einem Silikonkautschuk. Eine derartige Polsterschicht aus einem Silikonkautschuk paßt sich den anatomischen Verhältnissen des Fußes optimal an, wobei praktisch an allen Stellen des Fußes der gleiche spezifische Flächendruck entsteht. Eine Polsterschicht aus einem Silikonkautschuk soll ein hohes Federungsvermögen besitzen, was darauf zurückzuführen ist, daß der Silikonkautschuk bei Belastung senkrecht zur Schicht seitlich ausweicht und dadurch in benachbarten Bereichen eine Volumenvergrößerung erfährt, die in diesem Bereich stützend wirkt. Beim Gehen soll dadurch eine ständige leichte Massage der Fußfläche erzielt werden, die die Durchblutung des Fußes fördert. Aufgrund seiner elastischen Eigenschaften werden vom Silikonkautschuk Stöße und Erschütterungen abgefangen, wodurch die Belastung des Fußes, des Knies, des Hüftgelenkes und sogar der Wirbelsäule verringert wird. Diese bei Belastung der Polsterschicht aus Silikonkautschuk auftretende seitliche Materialverschiebung ist jedoch nur dann möglich, wenn die Polsterschicht auf einem Träger angeordnet ist.

Durch das DE-DM 88 00 116 ist ein Fersenkissen aus Silikonkautschuk mit einer Fersenbettung und gegebenenfalls einer randseitig daran anschließenden Schale bekannt, wobei das Fersenbett einen Bereich aus einem weicher eingestellten Silikonkautschuk aufweist. Dieser Bereich mit der Aufgabe eines Polsters aus einem weicher eingestellten Silikonkautschuk ist in dem härter eingestellten Silikonkautschuk des Fersenkissens eingebettet. Bei diesem Fersenkissen und bei allen Fällen, bei denen das Polster aus einem gegenüber dem Material der Einlegesohle oder des Fersenkissens weicher eingestellten Silikonkautschuk besteht, ergibt sich der Nachteil einer Bildung von Materialanhäufungen im Polsterrandbereich bei Belastung oder Einwirken eines Druckes auf das Polster, so wie dieser dem durch den Fersensporn auf das Polster ausgeübten Druck entspricht. Durch die Druckeinwirkung hat das Polstermaterial das Bestreben, seitlich auszuweichen und da die seitlichen Materialverschiebungen nicht vom Material der Einlage oder des Fußbettes aufgenommen bzw. absorbiert werden können, weil deren Material gegenüber dem Material des Polsters härter eingestellt ist, also einen höheren Shore-Härtegrad aufweist, hat dies zur Folge, daß es zu Materialanhäufungen im Polsterrandbereich in Form von wulstartigen Erhebungen oder wellenartigen Ausformungen kommt, die nicht zu einer Druckentlastung des Fußes beitragen, sondern auf die Fußsohle, den Fersensporn oder die Ferse des Fußes einen Druck ausüben, wodurch die eigentliche gewünschte orthopädische Wirkung beeinträchtigt wird und es somit zu Schmerzen kommt.

Demgegenüber ist es Aufgabe der vorliegenden Erfindung, eine orthopädische Einlage gemäß der eingangs beschriebenen Art zu schaffen, bei der nicht nur das Auftreten von unerwünschten hohen Druckbelastungen vermieden wird, sondern bei der auch Schmerz hervorrufende Materialverschiebungen am Polster bei Druckeinwirkung auf das Polster unterbunden werden. Bei durch auf das Polster einwirkende Belastung oder Druck auftretende Materialverschiebungen oder Materialstauchungen des Polsters sollen von diesem selbst aufgenommen und in der Einlage bzw. vom Fußbett absorbiert werden.

Gelöst wird diese Aufgabe bei einer derartigen orthopädischen Einlage durch die im Anspruch 1 angegebenen Merkmale.

Durch die erfindungsgemäße Ausgestaltung der orthopädischen Einlage mit einem Polster, dessen der Fußsohle abgekehrte Unterseite mit einer konkaven Einziehung versehen ist, werden bewegungsbedingte Belastungen auf Muskeln, Sehnen und Gelenke gedämpft, wodurch einem frühzeitigen Verschleiß vorgebeugt und schmerzhafte Zustände gelindert werden. Sowohl das Polster als auch die Einlage selbst, in die das Polster eingebettet ist, bestehen aus einem Kunststoff, insbesondere einem Silikonkautschuk bevorzugterweise gleicher Shore-Härte. Weichheit und Elastizität des Polstermaterials und des Einlagenmaterials sind gleich. Das Abfedern von Stoßbelastungen erfolgt über die spezielle Unterseitenausgestaltung des in die Einlage bzw. in das Fersenbett eingebetteten Polsters. Bei Belastung oder Druckanwendung auftretende Materialverschiebung beim Polster wird von der konkaven Ausnehmung an der Unterseite des Polsters aufgenommen, so daß es im Fußsohlen- bzw. Fußfersen- bzw. Fußsporn-Bereich zu keinen Materialanhäufungen kommt, wenn das Polster belastet oder ein Druck auf das Polster ausgeübt wird. Das seitlich ausweichende Material des Polsters wird in die konkave Ausnehmung an der Unterseite des Polsters verdrängt.

Mit der erfindungsgemäßen Einlage wird der Bewegungsapparat der unteren Extremitäten durch elastische Absorption harter Aufstöße, insbesondere im Fersenbereich, entlastet. Die Einlage trägt durch die Ausgestaltung des in ihr eingebetteten Polsters zu einer deutlichen Linderung schmerzhafter Zustände bei. Bei Anordnung des Polsters im Fersenbereich oder in einem Fersenbett wird der Fersensporn im kritischen Bereich punktuell druckentlastet. Es erfolgt eine Aktivierung müder Füße speziell bei einem Fersensporn. Partieller Fersendruck wird sofort entlastet. Die Einlage selbst kann zur Entlastung der Ferse bzw. des Fersensporns mit einem im Fersenbereich angeordneten Polster versehen sein. Die Länge der Einlage kann der Länge der Fußsohle entsprechen, jedoch sind auch Ausgestaltungen möglich, bei denen die Einlage Zweidrittel der Fußsohle abdeckt, aber auch Abmessungen der Einlage sind möglich, wenn diese ausschließlich als Fersenbett eingesetzt werden soll.

Nach einer ersten Ausführungsform der Erfindung ist das Polster in den Formkörper der Einlage so eingebettet, daß das Polster mit seiner Unterseite in der von der die Auflage der Einlage bildenden Unterseite liegenden Ebene angeordnet ist, wobei die Unterseite des Polsters mit einer konkaven Einziehung bzw. Ausnehmung versehen ist, wobei die Shore-Härte des Polstermaterials gleich oder ungleich der Shore-Härte des Einlagenmaterials ist.

Eine zweite Ausführungsform der Erfindung sieht eine orthopädische Einlage vor, bei der der polsterartige Bereich von einem Abschnitt in der Einlage gebildet ist, der auf der Unterseite der Einlage mit einer konkaven Einziehung bzw. Ausnehmung versehen ist.

Bei einer dritten Ausführungsform der Erfindung besteht das Polster aus einem weich-elastischen, gegenüber dem Material des Fußbettes oder des Fersenbettes härter eingestellten, inkompressiblen Silikonkautschuk und das Fußbett oder das Fersenbett zur Aufnahme einer bei Druckeinwirkung auf das Polster eintretenden seitlichen Materialverschiebung oder -ausdehnung aus einem kompressiblen Silikonkautschuk.

Vorteilhafte Ausgestaltungen der Erfindung sind in den Unteransprüchen gekennzeichnet.

Ausführungsbeispiele der Erfindung werden nachstehend anhand der Zeichnung näher erläutert. Es zeigen
Fig. 1 in einer schaubildlichen Ansicht eine als Fersenbett mit eingearbeitetem Polster ausgebildete orthopädische Einlage,
Fig. 2 einen senkrechten Schnitt gemäß Linie II-II in Fig.1,
Fig. 3 einen senkrechten Schnitt gemäß Linie II-II in Fig.1 einer weiteren Ausführungsform einer als Fersenbett mit eingearbeitetem Polster ausgebildeten orthopädischen Einlage,
Fig. 4 in einer schaubildlichen Ansicht eine als Fersenbett mit einem eingearbeiteten kreisförmigen Polster ausgebildete orthopädische Einlage,
Fig. 5 in einer schaubildlichen Ansicht eine als Fußbett mit zwei eingearbeiteten Polstern ausgebildete orthopädische Einlage,
Fig. 6 einen senkrechten Längsschnitt gemäß Linie VI-VI in Fig.5,
Fig. 7 in einer schaubildlichen Ansicht eine weitere Ausführungsform einer als Fersenbett mit ausgebildetem polsterartigem Bereich versehene orthopädische Einlage,

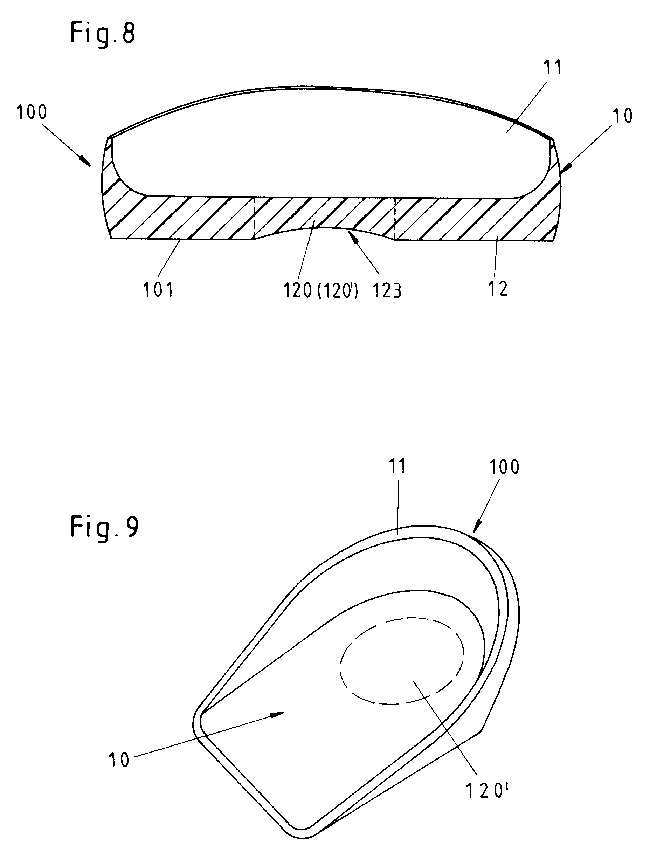

Fig. 8 einen senkrechten Schnitt gemäß Linie VIII-VIII in Fig.7,
Fig. 9 in einer schaubildlichen Ansicht eine als Fersenbett mit einem in diesem ausgebildeten polsterartigen Bereich ausgebildete orthopädische Einlage,
Fig.10 in einer schaubildlichen Ansicht eine als Fußbett mit zwei in diesem ausgebildeten polsterartigen Bereichen ausgebildete orthopädische Einlage,
Fig.11 einen senkrechten Längsschnitt gemäß Linie XI-XI in Fig.10,
Fig.12 in einer schaubildlichen Ansicht eine weitere Ausführungsform einer als Fersenbett mit eingearbeitetem Polster ausgebildete orthopädische Einlage,
Fig.13 in einem schematischen Querschnitt einen Abschnitt einer bekannten Einlage mit einem Polster, dessen Material gegenüber dem Material der Einlage weicher eingestellt ist, im druckunbelasteten Zustand des Polsters,
Fig.14 den Abschnitt der Einlage gemäß Fig.13, jedoch im druckbelasteten Zustand des Polsters,
Fig.15 einen senkrechten Schnitt gemäß Linie XV-XV in Fig.12,
Fig.16 in einem schematischen Querschnitt einen Abschnitt der Einlage gemäß Fig.12 mit einem Polster, dessen Material gegenüber dem Material der Einlage härter eingestellt ist,
Fig.17 in einer schaubildlichen Ansicht eine als Fersenbett mit einem eingearbeiteten kreisförmigen Polster ausgebildete orthopädische Einlage,
Fig.18 in einer schaubildlichen Ansicht eine als Fußbett mit zwei eingearbeiteten Polstern ausgebildete orthopädische Einlage und
Fig.19 einen senkrechten Längsschnitt gemäß Linie XIX-XIX in Fig.18.

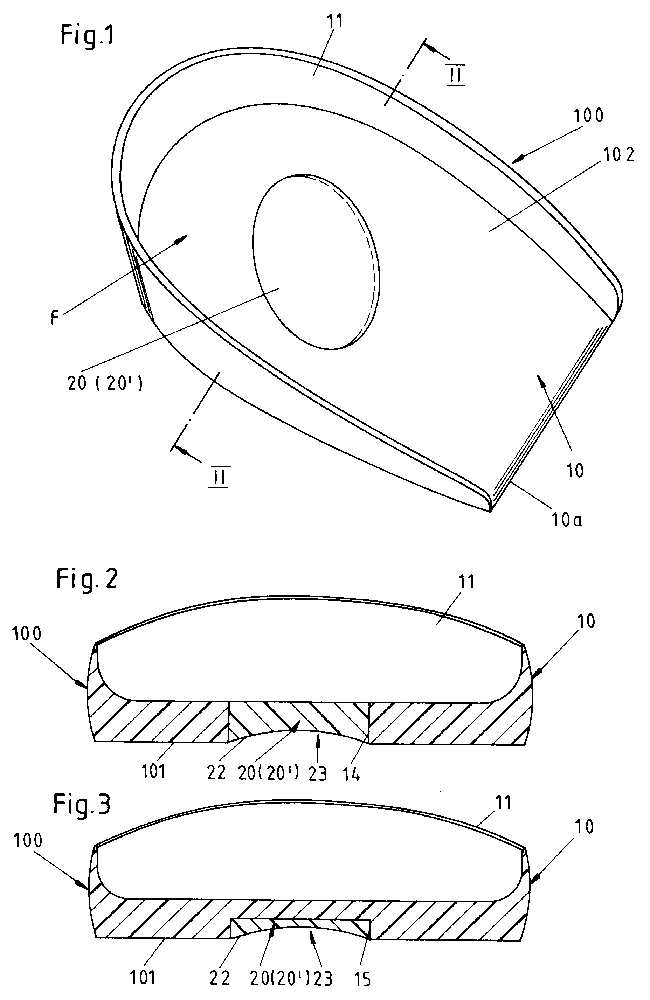

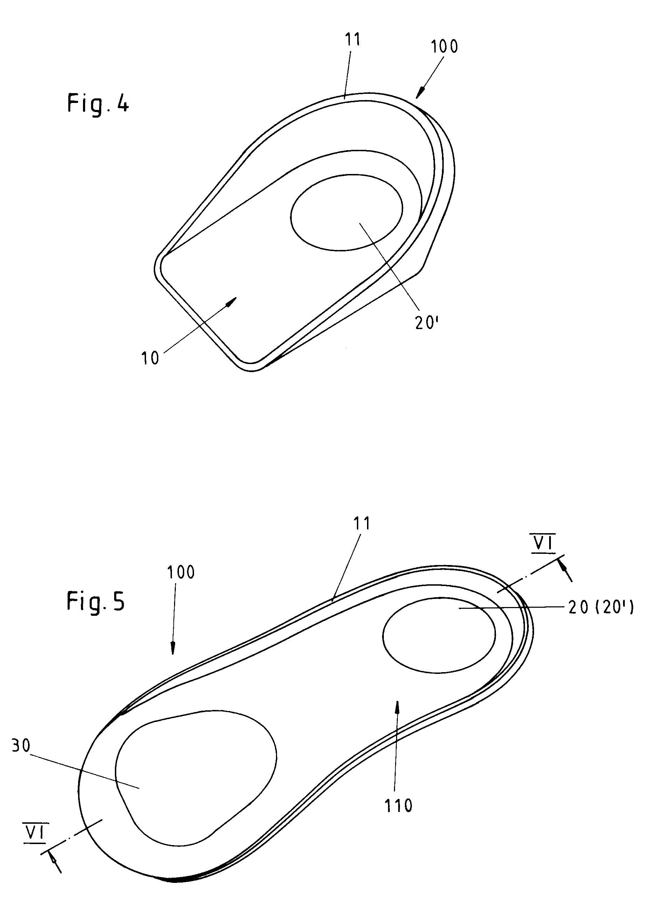

Die orthopädische Einlage 100 gemäß Fig.1 und 5 besteht aus einem Kunststoff, insbesondere einem Silikonkautschuk, wobei zweckmäßigerweise für die Herstellung der Einlage ein additionsvernetzter Silikonkautschuk verwendet wird. Diese Einlage 100 ist mit einer ebenen Unterseite 101 und einer ein Fußbett 110 (Fig.5) oder ein Fersenbett 10 (Fig. 1 und 4) bildenden, ausgearbeiteten Oberseite 102 versehen. Zweckmäßigerweise ist die Einlage 100 insbesondere im Fersenbereich F mit einem angeformten schalenförmigen Rand 11 versehen, der sich beim Fersenbett 10 bis zu dessen Vorderkante 10a und beim Fußbett 110 sich bis in den vorderen Bereich der Einlage erstreckt.

In die Einlage 100 ist mindestens ein Polster 20 bzw. 20' bzw. 30 im Bereich druckempfindlicher Stellen des Fußes eingearbeitet. Das im Fersenbereich F des Fersenbetts 10 bzw. des Fußbettes 110 vorgesehene Polster 20, 20' ist im Bereich des das Polster beaufschlagenden Fersensporns angeordnet. Nach einer weiteren Ausführungsform weist das Fußbett 110 der orthopädischen Einlage 100 im vorderen Zehenbereich bzw. im Bereich des Fußballens ein weiteres Polster 30 auf, um auch in diesem Bereich eine weich- bzw. federnd-elastische Abpolsterung des Fußes zu erreichen (Fig.5).

Auch das Polster 20,20' bzw. 30 besteht aus einem Kunststoff, insbesondere einem Silikonkautschuk, wobei auch hier zur Herstellung des Polsters ein additionsvernetzter Silikonkautschuk eingesetzt werden kann. Das Fersenbett 10 bzw. das Fußbett 110 der orthopädischen Einlage 100 und das Polster 20,20',30 bestehen somit aus gleichen Materialien, hier aus additionsvernetztem Silikonkautschuk. Dabei entspricht die Shore-Härte des Polstermaterials der Shore-Härte des Einlagenmaterials. Weder für das Polster wird ein gegenüber dem Einlagenmaterial weicher eingestelltes Material oder auch härter eingestelltes Material eingesetzt. Die Einlage 100 besteht somit entweder aus gleichem Material mit gleicher Shore-Härte oder aus ungleichem Material, wobei auch in diesem Fall beide Materialien gleiche Shore-Härte aufweisen.

So besteht z.B. die Möglichkeit, das Fersenbett 10 oder das Fußbett 110 aus einem Silikonkautschuk herzustellen, während das Polster aus einem Naturkautschuk oder einem geschlossenporigen Silikonschaum besteht, wobei jedoch beide Materialien gleiche Shore-Härte aufweisen.

Das Polster 20,20' bzw. 30 ist in die Einlage 100 bzw. in das Fersenbett 10 bzw. in das Fußbett 110 so eingearbeitet, daß das Polster bzw. die Polster mit ihrer Unterseite 22 in der von der die Auflage der Einlage 100 bildenden Unterseite 101 liegenden Ebene angeordnet sind. Die Unterseite 22 des Polsters 20,20' bzw. 30 ist dabei mit einer konkaven Einziehung 23 versehen, so daß zwischen dem Polster und der Fläche , auf der die orthopädische Einlage 100 aufliegt, ein Hohlraum ausgebildet ist, der zur Aufnahme von seitlich ausweichendem Material dient, wenn das Polster belastet bzw. auf das Polster ein Druck ausgeübt wird.

Für die Herstellung der Einlage 100 bzw. des Fersenbettes 10 oder des Fußbettes 110 und des Polsters 20, 20',30 werden weicheingestellte Kunststoffe, insbesondere Silikonkautschuke, eingesetzt, wobei ein Silikonkautschuk zweckmäßigerweise mit einer Härte von 2 bis 15 Shore eingesetzt werden kann.

Das Polster 20,20' zur Abstützung der Ferse und des Fersensporns ist als ovaler oder ellipsenförmiger Formkörper ausgebildet (Fig.1 und 4). Auch das Polster 30 im vorderen Fußbereich der orthopädischen Einlage 100 bzw. des Fußbettes 110 weist entsprechend Fig.5 eine Form auf, die einer in eine Ellipse übergehenden Kreisfläche entspricht. Die Polster 20,20' bzw. 30 können jedoch auch andere Formgebungen aufweisen.

Die Einlage 100 bzw. das Fersenbett 10 oder das Fußbett 110 bestehen aus einem glasklaren oder opaken federnd-elastischen Silikonkautschuk, wobei das Polster 20,20' bzw. 30 ebenfalls aus einem glasklaren oder opaken federnd-elastischen Silikonkautschuk bestehen kann. Bevorzugterweise wird jedoch das Polster 20,20' bzw. 30 aus einem eingefärbten federnd-elastischen Silikonkautschuk hergestellt, wobei diese Farbgebung des Polsters den Vorteil hat, daß, wenn das Polster oder Einlagen aus Materialien mit verschiedenen Shore-Härten eingesetzt werden, dann an der jeweiligen Farbgebung sogleich erkannt werden kann, ob eine Einlage 100 mit einem weicher oder härter eingestellten Kunststoff vorliegt. An der Farbgebung der Polster ist auch erkennbar, ob Einlagen aus einem weicher oder härter eingestellten Material vorliegen.

Das Polster 20,20' bzw. 30 ist in das Fersenbett 10 bzw. in das Fußbett 110 eingebettet. Nach einer ersten Ausführungsform gemäß Fig.2 weist die Einlage 100 eine das Polster 20,20' aufnehmende und der Form und den Abmessungen des Polsters entsprechende Durchbrechung 14 auf, in die das Polster 20,20' so eingebettet ist, daß ihre konkave Einziehung bzw. Ausnehmung 23 im Bereich der Unterseite 101 der Einlage zu liegen kommt.

Bei der Ausführungsform nach Fig.3 weist die Einlage 100 an ihrer Unterseite 101 eine der Form und den Abmessungen des Polsters 20,20' entsprechende Ausnehmung 15 auf, in die das Polster 20,20' so eingebettet ist, daß seine konkave Einziehung bzw. Ausnehmung 23 in der Ebene zu liegen kommt, die von der Unterseite der Einlage 100 gebildet wird.

Die Anzahl der Durchbrechungen 14 bzw. der Ausnehmungen 15 in der Einlage 100 entspricht der Anzahl der einzubettenden Polster. Auch das Polster 30 im vorderen Bereich des Fußbettes 110 der Einlage 100 entsprechend Fig.5 ist in gleicher Weise eingebettet wie das Polster 20,20'.

Das Einbetten des Polsters 20,20' bzw. in die Einlage 100 erfolgt in der Weise, daß zunächst das Fersenbett bzw. das Fußbett 110 hergestellt wird, wobei jedoch das verwendete Material, hier Silikonkautschuk, nicht ausvulkanisiert ist, was wesentlich ist, um eine Verwachsung mit dem Material des Polsters zu erreichen. In die vorgesehene Durchbrechung 14 bzw. Ausnehmung 15 wird dann eingefärbter Silikonkautschuk eingegossen, und zwar unter Ausbildung der konkaven Einziehung bzw. Ausnehmung 23 an der Unterseite 22 des Polsters 20,20', woraufhin dann das so hergestellte Produkt ausvulkanisiert wird. Auf diese Weise wird ein Verwachsen der Fußbett- bzw. Fersenbett-Materialien mit dem gleichen Material des Polsters erreicht.

Das Polster 20,20' bzw. 30 und die Einlage können auch aus Materialien unterschiedlicher Shore-Härte bestehen, wobei auch bei dieser Ausgestaltung das Polster auf seiner Unterseite mit der konkaven Einziehung 23 versehen ist.

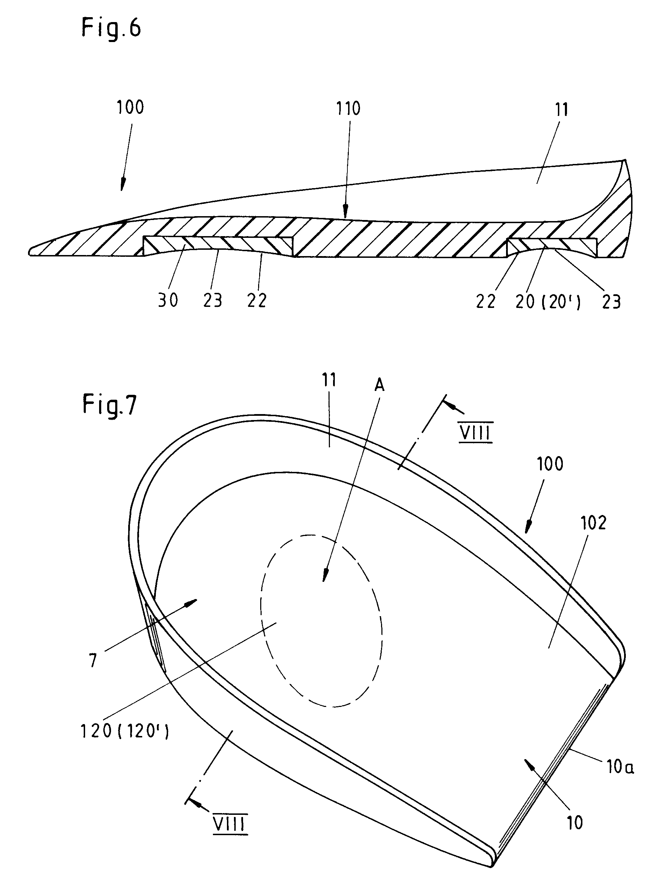

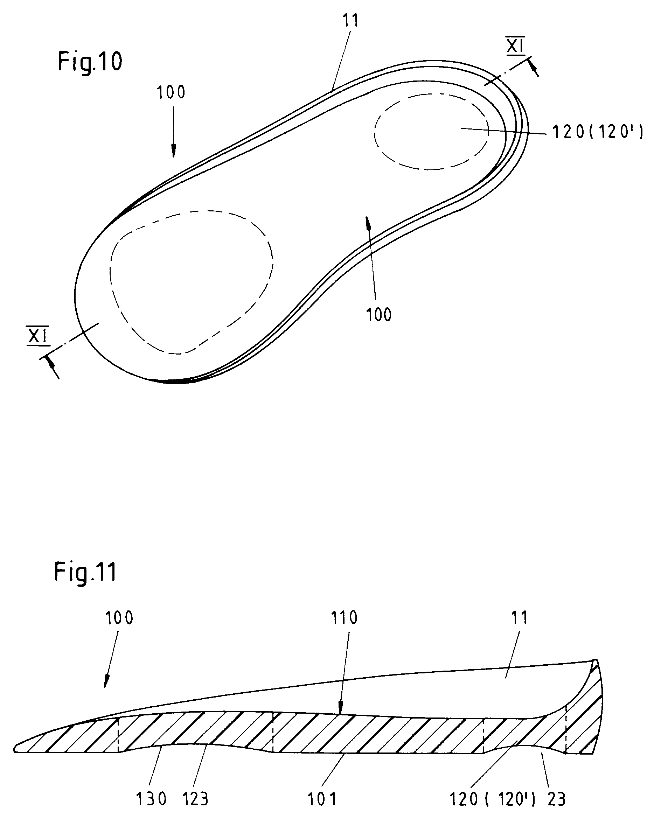

Die orthopädische Einlage 100 gemäß Fig.7 und 11 besteht ebenfalls aus einem Kunststoff, insbesondere einem Silikonkautschuk, wobei zweckmäßigerweise für die Herstellung der Einlage ein additionsvernetzter Silikonkautschuk verwendet wird.

Diese Einlage 100 ist mit einer ebenen Unterseite 101 und einer ein Fußbett 110 (Fig.11) oder ein Fersenbett 10 (Fig.7 und 10) bildenden, ausgearbeiteten Oberseite 102 versehen. Zweckmäßigerweise ist die Einlage 100 insbesondere im Fersenbereich F mit einem angeformten schalenförmigen Rand 11 versehen, der sich beim Fersenbett 10 bis zu dessen Vorderkante 10a und beim Fußbett 110 sich bis in den vorderen Bereich der Einlage erstreckt.

In der Einlage 100 ist mindestens ein Bereich bzw. Flächenabschnitte 120 bzw. 120'.bzw. 130 im Bereich druckempfindlicher Stellen des Fußes ausgebildet, der auf der Unterseite 101 der Einlage 100 mit einer konkaven Einziehnung bzw. Ausnehmung 123 versehen ist, die den Bereich begrenzt und vorgibt, der druckbeaufschlagt wird und als Polster wirkt. Der im Fersenbereich F des Fersenbetts 10 bzw. des Fußbettes 110 ausgebildete polsterartige Bereich 120,120' ist im Bereich des den polsterartigen Bereich oder Abschnitt der Einlage 100 das Polster beaufschlagenden Fersensporns angeordnet. Nach einer weiteren Ausführungsform weist das Fußbett 110 der orthopädischen Einlage 100 im vorderen Zehenbereich bzw. im Bereich des Fußballens einen weiteren polsterartigen Bereich 130 auf, um auch in diesem polsterartigen Bereich 130 eine weich- bzw. federnd-elastische Abpolsterung des Fußes zu erreichen (Fig.11).

Der polsterartige Bereich 120,120' bzw. 130 wird in der Einlage 100 bzw. in dem Fersenbett 10 bzw. in dem Fußbett 110 durch Ausbildung der Einziehung 123 an der Unterseite 101 der Einlage 100 erhalten. Die Unterseite 101 der Einlage 100 ist mit der konkaven Einziehung 123 versehen, so daß zwischen der Einlage 100 und der Fläche, auf der die orthopädische Einlage 100 aufliegt, ein Hohlraum ausgebildet ist, der zur Aufnahme von seitlich ausweichendem Material dient, wenn der polsterartige Bereich belastet bzw. wenn ein Druck ausgeübt wird.

Für die Herstellung der Einlage 100 bzw. des Fersenbettes 10 oder des Fußbettes 110 wird ein weicheingestellter Kunststoff, insbesondere ein Silikonkautschuk, eingesetzt, wobei ein Silikonkautschuk zweckmäßigerweise mit einer Härte von 2 bis 15 Shore verwendet wird.

Der polsterartige Bereich 120,120' zur Abstützung der Ferse und des Fersensporns weist eine ovale oder ellipsenförmige Form auf, wobei die Einziehung 123 die entsprechende Formgebung erhält, durch die der polsterartige Bereich 120,120' für die Abstützung des Fersensporns vorbestimmt, d.h. vorgegeben ist. Dieser polsterartige Bereich 120,120' ist in der Einlage 100 so ausgebildet, daß bei Anwendung der Einlage 100 der polsterartige Bereich unterhalb des Fersensporns zu liegen kommt bzw. dieser sich auf dem polsterartigen Bereich abstützt (Fig.7 und 10). Auch der polsterartige Bereich 130 im vorderen Fußbereich der orthopädischen Einlage 100 bzw. des Fußbettes 110 weist entsprechend Fig.11 eine Form auf, die einer in eine Ellipse übergehenden Kreisfläche entspricht. Die polsterartigen Bereiche 120,120' bzw. 130 können jedoch auch andere Formgebungen aufweisen.

Die Einlage 100 bzw. das Fersenbett 10 oder das Fußbett 110 bestehen aus einem glasklaren oder opaken federnd-elastischen Silikonkautschuk. Bei der Herstellung der Einlage 100 kann der Einlagenabschnitt eingefärbt werden, in dem der polsterartige Bereich 120,120',130 ausgebildet wird. Diese Farbgebung des Polsters hat den Vorteil, daß wenn Einlagen aus Materialien unterschiedlicher Shore-Härte eingesetzt werden, dann an der jeweiligen Farbgebung sogleich erkannt werden kann, ob eine Einlage 100 mit einem weicher oder härter eingestellten Kunststoff vorliegt.

Die Formgebung und Ausbildung der Einziehung 123 erfolgt bei der Herstellung der Einlage 100.

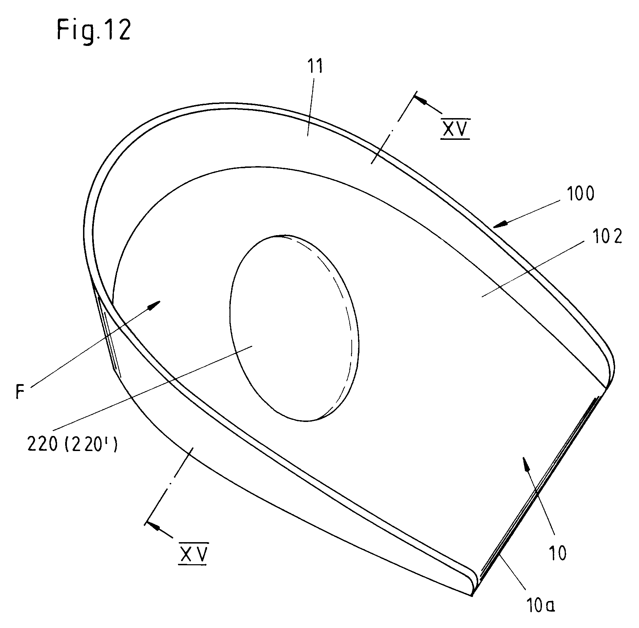

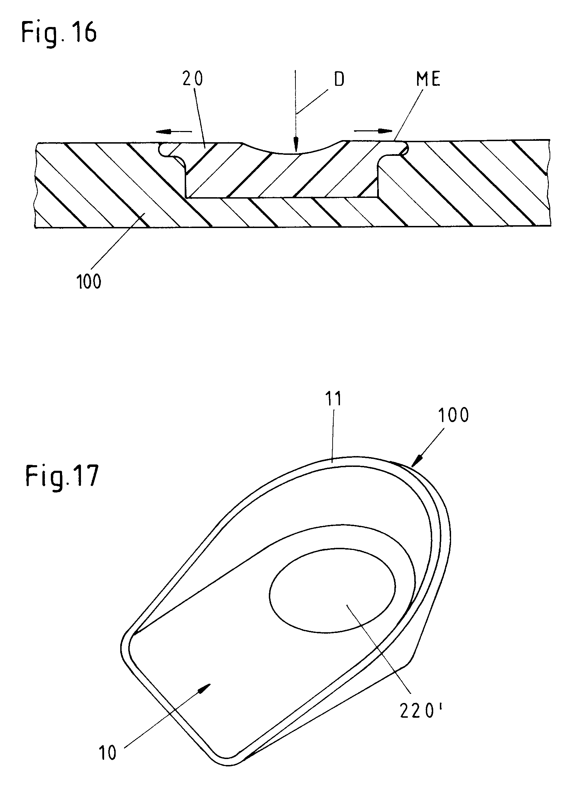

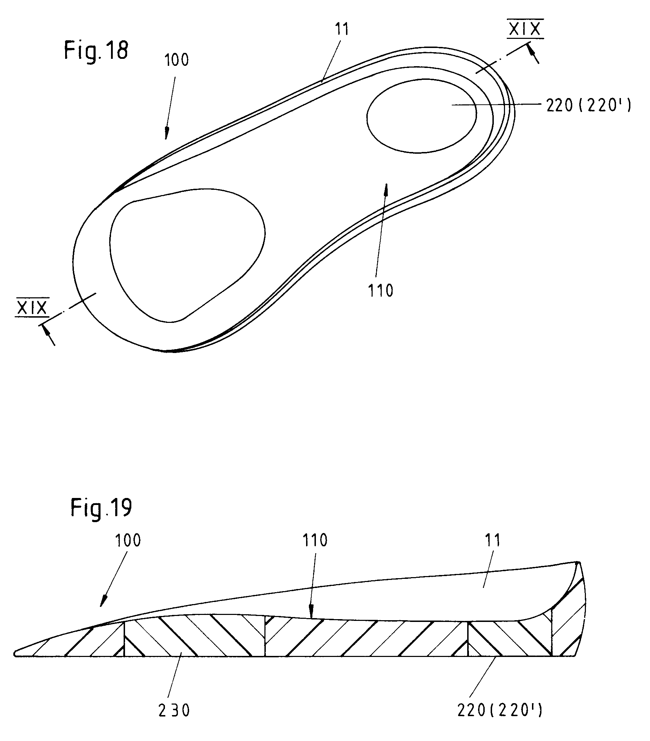

Die orthopädische Einlage 100 gemäß Fig.12 besteht ebenfalls aus einem Kunststoff, insbesondere einem Silikonkautschuk, wobei zweckmäßigerweise für die Herstellung der Einlage ein additionsvernetzter Silikonkautschuk verwendet wird. Diese Einlage 100 ist mit einer ebenen Unterseite 101 und einer ein Fußbett 110 (Fig.18 und 19) oder ein Fersenbett 10 (Fig.12 und 17) bildenden, ausgearbeiteten Oberseite 102 versehen. Zweckmäßigerweise ist die Einlage 100 insbesondere im Fersenbereich F mit einem angeformten schalenförmigen Rand 11 versehen, der sich beim Fersenbett 10 bis zu dessen Vorderkante 10a und beim Fußbett 110 sich bis in den vorderen Bereich der Einlage erstreckt.

In die Einlage 100 ist mindestens ein Polster 220 bzw. 220' bzw. 230 im Bereich druckempfindlicher Stellen des Fußes eingearbeitet. Das im Fersenbereich F des Fersenbetts 10 bzw. des Fußbettes 110 vorgesehene Polster 220, 220' ist im Bereich des das Polster beaufschlagenden Fersensporns angeordnet, d.h. bei Gebrauch der Einlage liegt das Polster 220,220' unterhalb des Fersensporns. Nach einer weiteren Ausführungsform weist das Fußbett 110 der orthopädischen Einlage 100 im vorderen Zehenbereich bzw. im Bereich des Fußballens ein weiteres Polster 230 auf, um auch in diesem Bereich eine weich- bzw. federnd-elastische Abpolsterung des Fußes zu erreichen (Fig.18 und 19).

Auch das Polster 220,220' bzw. 230 besteht aus einem Kunststoff, insbesondere einem Silikonkautschuk, wobei auch hier zur Herstellung des Polsters ein additionsvernetzter Silikonkautschuk eingesetzt werden kann. Das Fersenbett 10 bzw. das Fußbett 110 der orthopädischen Einlage 100 und das Polster 220,220',230 bestehen somit aus gleichen Materialien, hier aus additionsvernetztem Silikonkautschuk. Dabei ist die Shore-Härte des Polstermaterials gegenüber der Shore-Härte des Einlagenmaterials etwas größer.

Das Polster 220,220',230 besteht bevorzugterweise aus einem weich-elastischen, gegenüber dem Material der Einlage 100 bzw. des Fußbettes 110 bzw. des Fersenbettes 10 härter eingestellten inkompressiblen Silikonkautschuk, während das Fußbett bzw. das Fersenbett, d.h. die das Polster aufnehmende Einlage 100, zur Aufnahme einer bei Druckeinwirkung auf das Polster 220,220',230 eintretenden seitlichen Materialverschiebung oder -ausdehnung aus einem kompressiblen Silikonkautschuk gefertigt ist, wobei auch andere geeignete, gleiche Wirkungen aufweisende Kunststoffe eingesetzt werden können.

Das Material des Fußbettes 110 bzw. des Fersenbettes 10 weist eine gegenüber der Shore-Härte des Materials des Polsters 220,220',230 größere Shore-Härte auf, wobei die Shore-Härte für das Material des Fußbettes 110 bzw. des Fersenbettes 10 etwa zwei bis fünfzehn beträgt, während das Material des Polsters 220,220',230 eine über zwei bis fünfzehn Shore-Härte liegende Shore-Härte aufweist.

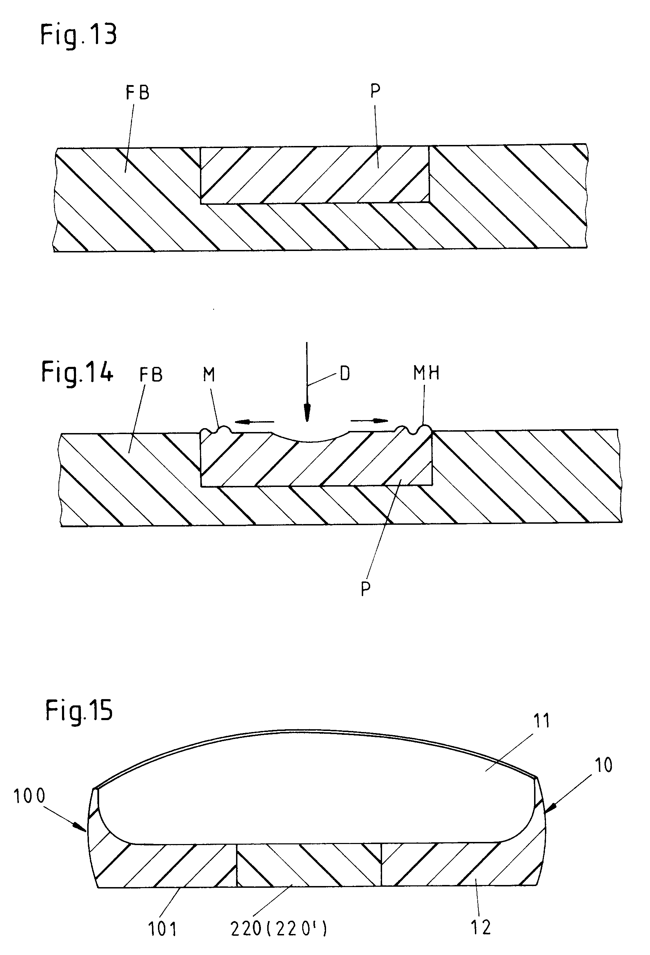

Damit wird folgender Vorteil erreicht:
Da bei einem bekannten Fußbett FB gemäß Fig.13 und 14 das Polster P gegenüber dem Material des Fußbettes weicher eingestellt ist, kann bei einer Einwirkung eines Druckes D auf das Polster P das sich seitlich verschiebende Material M nicht vom Fußbett FB aus einem härteren Material aufgenommen werden; das härtere Material des Fußbettes bremst eine seitliche Materialverschiebung des Polsters ab mit der Folge, daß es im Grenzbereich Polster/Fußbett zu Materialanhäufungen MH kommt, die einen unerwünschten Druck auf die Fußferse ausüben (Fig.14).

Ist dagegen erfindungsgemäß das Polstermaterial gegenüber dem Einlagenmaterial härter eingestellt, dann kann es bei einer Druckeinwirkung auf das Polster 220 nicht zu Materialanhäufungen im Randbereich des Polsters 220 zur Einlage 100 kommen, sondern das sich seitlich verschiebende Material des Polsters 220 wird vom kompressiblen Material der Einlage aufgenommen, wobei letzteres bei ME komprimiert wird (Fig.16).

Für die Herstellung der Einlage 100 bzw. des Fersenbettes 10 oder des Fußbettes 110 und des Polsters 220, 220',230 werden weicheingestellte Kunststoffe, insbesondere Silikonkautschuke, eingesetzt, wobei ein Silikonkautschuk zweckmäßigerweise mit einer Härte von zwei bis fünfzehn Shore für das Polster eingesetzt werden kann, wohingegen das Material der Einlage 100 eine über dieser Shore-Härte liegende Shore-Härte aufweist.

Das Polster 220,220' zur Abstützung der Ferse und des Fersensporns ist als ovaler, ellipsenförmiger oder kreisförmiger Formkörper ausgebildet (Fig.12 und 17). Auch das Polster 230 im vorderen Fußbereich der orthopädischen Einlage 100 bzw. des Fußbettes 110 weist entsprechend Fig.12 eine Form auf, die einer in eine Ellipse übergehenden Kreisfläche entspricht. Die Polster 220,220' bzw. 230 können jedoch auch andere Formgebungen aufweisen.

Die Einlage 100 bzw. das Fersenbett 10 oder das Fußbett 110 bestehen aus einem glasklaren oder opaken federnd-elastischen Silikonkautschuk, wobei das Polster 220,220' bzw. 230 ebenfalls aus einem glasklaren oder opaken federnd-elastischen Silikonkautschuk bestehen kann. Bevorzugterweise wird jedoch das Polster 220,220' bzw. 230 aus einem eingefärbten federnd-elastischen Silikonkautschuk hergestellt, wobei diese Farbgebung des Polsters den Vorteil hat, daß, wenn Polster oder Einlagen aus Materialien mit verschiedenen Shore-Härten eingesetzt werden, dann an der jeweiligen Farbgebung sogleich erkannt werden kann, ob eine Einlage 100 mit einem weicher oder härter eingestellten Kunststoff vorliegt. An der Farbgebung der Polster ist auch erkennbar, ob Polster aus einem weicher oder härter eingestellten Material vorliegen.

Das Einbetten des Polsters 220,220' bzw. in die Einlage 100 erfolgt in der Weise, daß zunächst das Fersenbett bzw. das Fußbett 110 hergestellt wird, wobei jedoch das verwendete Material, hier Silikonkautschuk, nicht ausvulkanisiert ist, was wesentlich ist, um eine Verwachsung mit dem Material des Polsters zu erreichen. In eine ausgesparte, das Polster aufnehmende Durchbrechung bzw. Ausnehmung wird dann eingefärbter Silikonkautschuk eingegossen, woraufhin dann das so hergestellte Produkt ausvulkanisiert wird. Auf diese Weise wird ein Verwachsen der Fußbett- bzw. Fersenbett-Materialien mit dem gleichen Material des Polsters erreicht.

Das Polster 220,220',230 ist entweder in eine Ausnehmung in dem Formkörper der Einlage 100 von oben (Fig.16), von unten oder in einer in der Einlage 100 ausgebildeten Durchbrechung eingesetzt (Fig.15 und 19).

## Patentansprüche

1. Orthopädische Einlage (100) mit einer ebenen Unterseite (101) und einer ein Fußbett (110) oder Fersenbett (10) bildenden, ausgearbeiteten Oberseite (102) mit mindestens einem eingearbeiteten Polster (20;20'; 30) im Bereich druckempfindlicher Stellen des Fußes, insbesondere des Fersensporns, wobei die Einlage (100) und das Polster (20;20';30) aus einem Kunststoff, insbesondere einem Silikonkautschuk, bestehen,
dadurch gekennzeichnet,
daß das Polster (20,20',30;120,120',130;220,220',230) durch Ausbildung eines Bereichs mit einer in der Unterseite der Einlage (100) in dem das Polster bildenden Bereich ausgebildeten konkaven Einziehung bzw. Ausnehmung (23,123) in dem Formkörper der Einlage (100) erhalten wird, wobei dieser polsterartige Bereich aus einem Material mit einer Shore-Härte besteht, die gleich oder ungleich der Shore-Härte des Einlagenmaterials ist.

2. Einlage nach Anspruch 1,
dadurch gekennzeichnet,
daß das Polster (20;20';30) in den Formkörper der Einlage (100) so eingebettet ist, daß das Polster mit seiner Unterseite (22) in der von der die auflage der Einlage (100) bildenden Unterseite (101) liegenden Ebene angeordnet ist, und daß die Unterseite (22) des Polsters (20;20';30) mit einer konkaven Einziehung bzw. Ausnehmung (23) versehen ist, wobei die Shore-Härte des Polstermaterials gleich oder ungleich der Shore-Härte des Einlagenmaterials ist.

3. Einlage nach Anspruch 1,
dadurch gekennzeichnet,
daß der polsterartige Bereich (120,120',130) von einem Abschnitt (A) in der Einlage (100) gebildet ist, der auf der Unterseite (101) der Einlage (100) mit einer konkaven Einziehung bzw. Ausnehmung (123) versehen ist.

4. Einlage nach Anspruch 1,
dadurch gekennzeichnet,
daß das Polster bzw. der polsterartige Bereich (220, 220',230) aus einem weich-elastischen, gegenüber dem Material des Fußbettes (110) oder des Fersenbettes (10) härter eingestellten, inkompressiblen Silikonkautschuk und das Fußbett (110) oder das Fersenbett (10) zur Aufnahme einer bei Druckeinwirkung auf das Polster (220,220',230) eintretenden seitlichen Materialverschiebung oder -ausnehmung aus einem kompressiblen Silikonkautschuk besteht.

5. Einlage nach einem der Ansprüche 1 und 2,
dadurch gekennzeichnet,
daß die Einlage (100) eine das Polster (20;20';30) aufnehmende und der Form und den Abmessungen des Polsters entsprechende Durchbrechung (14) aufweist, in die das Polster eingebettet ist.

6. Einlage nach einem der Ansprüche 1 bis 5,
dadurch gekennzeichnet,
daß die Einlage (100) an ihrer Unterseite (101) eine der Form und den Abmessungen des Polsters (20;20';30) entsprechende Ausnehmung (15) aufweist, in die das Polster eingebettet ist.

7. Einlage nach einem der Ansprüche 1,2,5 und 6,
dadurch gekennzeichnet,
daß die Einlage (100) eine der Anzahl der eingebetteten Polster entsprechende Anzahl von Durchbrechungen (14) oder Ausnehmungen (15) aufweist.

8. Einlage nach einem der Ansprüche 1 und 3,
dadurch gekennzeichnet,
daß die Einlage (100) mehrere polsterartige Abschnitte (A) mit auf der Unterseite (101) der Einlage ausgebildeten konkaven Einziehungen bzw. Ausnehmungen (123) aufweist.

9. Einlage nach einem der Ansprüche 1 bis 8,
dadurch gekennzeichnet,
daß die Einlage (100) bzw. das Fersenbett (10) bzw. das Fußbett (110) aus einem glasklaren oder opaken, federnd-elastischen Silikonkautschuk und das Polster (20,20',30;120,120',130;220,220',230) aus einem glasklaren oder opaken, federnd-elastischen Silikonkautschuk oder aus einem mit einer Farbgebung versehenen federnd-elastischen Silikonkautschuk bestehen.

10. Einlage nach einem der Ansprüche 1 bis 9,
dadurch gekennzeichnet,
daß die Einlage (100) und das Polster (20,20',30; 120,120',130;220,220',230) aus einem additionsvernetzten Silikonkautschuk bestehen.

11. Einlage nach einem der Ansprüche 1 bis 10,
dadurch gekennzeichnet,
daß die Einlage (100) und das Polster (20,20',30; 120,120',130;220,220',230) aus einem Silikonkautschuk bestehen, der eine Härte von zwei bis fünfzehn Shore aufweist.

12. Einlage nach einem der Ansprüche 1 bis 11,
dadurch gekennzeichnet,
daß das zur Abstützung des Fersensporns dienende Polster (20,20';120,120';220,220') als ovaler oder ellipsenförmiger Formkörper ausgebildet ist.

13. Einlage nach einem der Ansprüche 1 bis 12,
dadurch gekennzeichnet,
daß die Einlage (100) in ihrem Fersenbereich mit einem schalenförmigen, sich bis in den vorderen, dem Fersenbereich abgekehrten Bereich verlaufenden Rand (11) versehen ist.

14. Einlage nach einem der Ansprüche 1 bis 13,
dadurch gekennzeichnet,
daß das Polster oder der den polsterartigen Bereich (20,20',30;120,120'130;220,220',230) bildende Abschnitt (A) der Einlage (100) mit einer Farbgebung versehen ist.

15. Einlage nach einem der Ansprüche 1 bis 14,
dadurch gekennzeichnet,
daß das Material des Fußbettes (110) bzw. des Fersenbettes (10) eine gegenüber der Shore-Härte des Materials des Polsters (20,20'30;120,120'130;220,220, 230) größere Shore-Härte aufweist.

16. Einlage nach einem der Ansprüche 1 bis 15,
dadurch gekennzeichnet,
daß das Material des Fußbettes (110) bzw. des Fersenbettes (10) eine Härte von zwei bis fünfzehn Shore und das Material des Polsters (20,20',30;120,120',130; 220,220',230) eine über zwei bis fünfzehn Shore-Härte liegende Shore-Härte aufweist.
